Europäisches Patentamt

European Patent Office

Office européen des brevets

(19)

(11) Numéro de publication: **0 079 595**
**B1**

(12) **FASCICULE DE BREVET EUROPEEN**

(45) Date de publication du fascicule du brevet:
17.04.85

(51) Int. Cl.⁴: **C 12 N 11/10, C 12 N 11/14**

(21) Numéro de dépôt: 82110446.0

(22) Date de dépôt: 12.11.82

(54) Procédé de préparation de biocatalyseurs doués d'activité enzymatique et produits obtenus.

(30) Priorité 17.11.81 CH 7375/81

(43) Date de publication de la demande:
25.05.83 Bulletin 83/21

(45) Mention de la délivrance du brevet:
17.04.85 Bulletin 85/16

(84) Etats contractants désignés:
BE DE FR GB IT NL SE

(73) Titulaire: SOCIETE DES PRODUITS NESTLE S.A., Case postale 353, CH-1800 Vevey (CH)

(72) Inventeur: Leuba, Jean-Louis, 1049 Boussens (CH)
Inventeur: Renken, Albert, Chemin des Charmilles 46, 1025 Saint-Sulpice (CH)
Inventeur: Flaschel, Erwin, Route Praz-Veguey 20, 1022 Chavannes (CH)

(56) Documents cités
WO - A - 82/00660
FR - A - 2 306 997
US - A - 3 909 358
US - A - 4 089 746
US - A - 4 167 447

CHEMICAL ABSTRACTS, vol. 83, no. 2, 14 juillet 1975, page 126, no. 12563n, Columbus Ohio (USA); C.G. ALLAN et al.: "Marine polymers. V. Modification of paper with partially deacetylated chitin"
CHEMICAL ABSTRACTS, vol. 91, no. 1, 2 juillet 1979, page 203, no. 1923u, Columbus Ohio (USA); M.S. MASRI et al.: "Insolubilizing enzymers with chitosan and chitosan-derived polymers"
CHEMICAL ABSTRACTS, vol. 90, no. 17, 23 avril 1979, page 223, no. 134540t, Columbus Ohio (USA); F.

(56) Documents cités: (suite)
WIDMER: "Chitosan: a promising support for enzyme immobilization. Preliminary system design and assessment with Aspergillus niger beta-galactosidase (lactase)"
CHEMICAL ABSTRACTS, vol. 89, no. 3, 17 juillet 1978, page 254, no. 19193z, Columbus Ohio (USA); F. BISSETT et al.: "Immobilization of aspergillus beta-glucosidase on chitosan"
AGRIC. BIOL. CHEM., vol. 41, no. 10, 1977, pages 1865-1872; T. KASUMI et al.: "Preparation and some properties of chitosan bound enzymes"

## Description

La présente invention a trait à un procédé de préparation de biocatalyseurs doués d'activité enzymatique insolubles en milieux aqueux et convenant particulièrement à l'utilisation en lit fluidisé ou en lit fixe.

La mise en œuvre industrielle de réactions enzymatiques a fait apparaître des biocatalyseurs insolubles en milieux aqueux. En effet, les enzymes sont trop précieux pour être désactivés après une seule utilisation, ce qui a conduit à les fixer sur un support inerte ou à les »immobiliser« de manière à pouvoir les séparer physiquement de la substance à traiter ou »substrat«.

On a proposé plusieurs méthodes de fixation d'enzymes sur des supports:

— L'enzyme est adsorbé directement sur un matériau minéral tel que la céramique, le verre, la silice, etc... Les produits obtenus ont l'inconvénient d'être peu stables, l'enzyme n'étant que faiblement lié au support.

— L'enzyme est lié chimiquement à un support organique tel qu'un polymère synthétique (par exemple un amino éthyl cellulose selon le brevet britannique No. 1 357 317) ou naturel (par exemple le chitosane selon le brevet des Etats-Unis No. 4 094 743) par l'intermédiaire d'un réactif bi-fonctionnel tel qu'un dialdéhyde ou monofonctionnel comme une carbodiimide. Les enzymes immobilisés sur un support synthétique n'ont pas la qualité alimentaire requise. D'autre part, les supports organiques ne sont pas bient adaptés aux procédés industriels car ils sont compressibles et d'un poids spécifique relativement faible et de ce fait leur utilisation dans des réacteurs à lit fluidisé ou à lit fixe à des débits élevés est difficile sinon impossible.

En outre, il est nécessaire d'assurer un bon contact entre l'enzyme et la substance à convertir ou substrat. Cela implique que l'enzyme soit immobilisé de préférence sur la surface extérieure et pas dans les pores ou dans la matrice du support inerte.

Les difficultés et limites de l'application mentionnées sont plus sévères si le milieu réactionnel est visqueux et s'il contient des substances ou impuretés colloïdales ou solides. De telles difficultés sont rencontrées couramment dans le domaine de l'industrie alimentaire.

Nous avons trouvé une méthode d'immobilisation d'enzyme avantageuse, compatible avec une application dans l'industrie alimentaire et qui ne présente pas les inconvénients précités.

Le procédé selon l'invention est caractérisé par le fait qu'on enrobe des particules d'une matière minérale rigide et dense avec une couche de chitosane, qu'on stabilise les particules enrobées par un réactif bi-fonctionnel et qu'on fixe un enzyme sur le support ainsi traité.

Le noyau minéral utilisé est choisi parmi les substances minérales inertes chimiquement rigides et de préférence poreuses en surface. On peut citer par exemple les particules microporeuses de gel de silice, d'oxydes métalliques tels que $TiO_2$, $ZrO_2$ de céramique, etc... Les particules ont généralement 0,05 à 5 mm de diamètre, et de préférence 0,1 à 0,25 mm.

Pour l'enrobage du noyau, on utilise du chitosane qui est un polymère naturel obtenu par deacétylation de la chitine en milieu fortement basique et à température élevée. Il est insoluble dans l'eau, sauf en présence d'un acide autre que l'acide sulfurique.

La chitine est le composant organique principal de l'enveloppe protectrice des invertébrés (carapace de crustacés ou cuticule d'insectes) que l'on trouve aussi dans la paroi squelettique de certaines formes inférieures végétales (champignons par exemple). C'est un polymère linéaire de la N-acétyl-D-glucosamine, de haut poids moléculaire (200 000 environ), qui se caractérise par des liaisons du type $\beta$ (1→4). Le groupe N-acétyl étant difficile à éliminer, la déacétylation n'est jamais complète et varie généralement de 50 à 85% dans les produits commerciaux. Ce sont ces produits que l'on désigne sous le nom de chitosane.

Pour charger la surface des particules minérales avec du chitosane, on prépare une solution aqueuse de chitosane dans un acide, par exemple l'acide acétique concentré (10 à 80% en poids et de préférence 30 à 60%), à raison de 1 à 30 g de chitosane/l, et de préférence 4 à 10 g/l. On ajoute à la solution les particules minérales sous agitation jusqu'à l'obtention d'une concentration de 10 à 500 g de matière solide par litre. On dégaze le mélange sous vide, laisse la suspension au repos quelques heures et, après décantation, on récupère les particules enrobées par filtration. Celles-ci sont lavées à l'eau et séchées sous vide. Le support ainsi obtenu a pratiquement la masse volumique du support minéral, par exemple de 0,33 g/ml pour le gel de silice, c'est-à-dire plus élevée que celle du chitosane (0,24 g/ml). Il a par rapport au chitosane seul des avantages décisifs du point de vue de l'utilisation industrielle. En effet:

— Il est généralement aussi actif en unités enzymatiques par gramme de poids sec que le chitosane seul. Ceci s'explique par le fait que les sites actifs du chitosane sont mieux utilisés puisqu'ils sont répartis sur la surface des particules.

— Du fait de sa masse volumique plus élevée, il occupe dans une colonne un volume plus faible que le chitosane pour un poids ou une activité équivalents.

— Le noyau minéral rend le catalyseur beaucoup plus dur et rigide, donc moins compressible que le chitosane.

— Le catalyseur est adapté à une utilisation en lit fluidisé qui est compatible avec une utilisation industrielle. Lors de la fluidisation, le substrat peut être transformé en utilisant des débits élevées. Le traitement des fluides biologiques (ex. lait, sérum lactique, etc.) ou des fluides contenant des colloïdes en suspension posent beaucoup moins de problèmes de colmatage qu'en lit fixe. Des essais de fluidisation sur chitosane ont été réalisés mais n'ont pas donné satisfaction. Il se forme des aggrégats et du »channelling«. L'utilisation du chitosane en lit fixe pose aussi quelques problèmes; lorsque le débit augmente, le chitosane de par sa faible densité, a tendance à se soulever et à se tasser à la sortie de la colonne. La pression interne augmente et le processus s'amplifie avec diminution rapide du débit et colmatage de la colonne. Un autre avantage de la fluidisation est que les particules de catalyseur sont en agitation permanente dans le substrat. De ce fait, les problèmes de diffusion sont limités et l'activité enzymatique est maximale.

— Les manipulations sont beaucoup plus aisées qu'avec le chitosane.

Les particules enrobées de chitosane sont traitées avec un réactif bi-fonctionnel de préférence choisi parmi les dialdéhydes linéaires. Ce peut être par exemple le malonaldéhyde, le succinaldéhyde, le glutaraldéhyde, l'adipaldéhyde. On préfère utiliser le glutaraldéhyde qui présente l'avantage d'être un produit commercial facilement disponible. Cette opération a pour but »d'activer« le chitosane et/ou de former un réseau de polymère autour de la couche de chitosane de manière à la fixer au noyau minéral et éviter le pelage de celle-ci.

Selon une première forme d'exécution du procédé, utilisable lorsque l'enzyme n'est pas sensible au glutaraldéhyde, c'est-à-dire lorsqu'il ne perd pas ses propriétés catalytiques en présence de glutaraldéhyde, le support est mis en présence d'un solution aqueuse de glutaraldéhyde de concentration allant jusqu'à 25% en poids. Comme enzymes insensibles au glutaraldéhyde, on peut citer ceux qui n'ont pas de groupe thiol dans leur site actif. Le traitement s'effectue de préférence à température ambiante à pH compris entre 2 et 8, de préférence dans une solution tampon. La durée du traitement dépend de la concentration en glutaraldéhyde. Elle est généralement comprise entre 31 min et quelques heures. On laisse réagir de préférence en l'absence d'air, par exemple sous vide et on élimine l'excès de glutaraldéhyde par lavage à l'eau. La concentration et la durée de la réaction sont telles que la surface du support soit hérissée de groupes aldéhydes liés chimiquement à celui-ci, le glutaraldéhyde fixé représentant, par exemple dans le cas où le support est un gel de silice, de 0,5 à 5% du poids du support. Le support est ainsi activé.

On fixe alors l'enzyme contenant des groupes fonctionnels capables de réagir acec les groupes aldéhydes libres du support activé, par exemple les groupes amino libres portés par les maillons lysine, par mise en contact du support activé avec une solution aqueuse de l'enzyme. Il est nécessaire de maintenir le pH de la solution enzymatique dans le domaine de stabilité de l'enzyme mis en œuvre, par exemple au moyen d'une solution tampon appropriée. La réaction a lieu habituellement à température ambiante, le maintien d'une température basse n'étant nécessaire que dans le cas des enzymes fragiles. L'enzyme non fixé est ensuite éliminé par lavage avec une solution tampon. Pour obtenir un catalyseur particulièrement stable, ce qui est préféré pour l'utilisation en lit fluidisé, on peut effectuer un traitement complémentaire du support déjà chargé de l'enzyme avec un réactif bi-fonctionnel, de préférence le glutaraldéhyde, par exemple en solution aqueuse de concentration allant jusqu'à 25% en poids. Ce traitement complémentaire a pour effet de réticuler l'ensemble et également de renforcer la liaison entre l'enzyme et le support.

Une seconde forme d'exécution du procédé est mise en œuvre dans le cas où l'enzyme perd ses propriétés catalytiques en présence de glutaraldéhyde, par exemple dans le cas d'une lactase de levure possédant des groupes thiols sensibles aux oxydants. On traite le support enrobé du chitosane et réticulé comme indiqué ci-dessus par mise en présence d'une solution contenant de 0,01 à 1% en poids de glutaraldéhyde, par réaction des groupes aldéhydes libres avec un composé porteur d'un groupe amine comme la glycine, l'asparagine ou la lysine ou, de préférence, par réduction de ceux-ci par exemple à l'aide de borohydrure de sodium. Après inactivation des groupes aldéhydes, on met le support en présence d'un enzyme, par exemple d'une lactase de levure, préalablement protégé au niveau du site actif par un composé inhibiteur compétitif tel que par exemple la galactono-$\gamma$-lactone ou la glucono-$\gamma$-lactone à la concentration de 20 à 50 mg/ml de milieu réactionnel. Ce sucre protecteur ne modifie pas l'adsorption de l'enzyme sur le support et est libéré par un excès de lactose. Le traitement a lieu dans une solution tampon adéquate en général à basse température par exemple de 2 à 10°C pendant 30 min à 24 heures, et l'excès d'enzyme fixé est lavé avec une solution tampon puis à l'eau.

Pour augmenter la stabilité de l'enzyme, on préfère lier chimiquement celui-ci au chitosane par l'intermédiaire d'un agent de couplage de préférence un carbodiimide ou le réactif de Woodward, 3-(2-éthyl-5-isoxazolo) benzène sulfonate, capable d'activer les groupes carboxyles de l'enzyme et de les faire réagir avec les groupes amino libres du chitosane. La réaction a lieu pendant 1 à 48 heures à température de 2 à 25°C en maintenant un pH de 4 à 6,5, en utilisant de 10 à 50 mg de carbodiimide par ml de milieu réactionnel. Une fois la réaction achevée, on lave ensuite l'excès de réactif et l'urée produite par la réaction avec une solution tampon appropriée puis à l'eau. En variante, au lieu de procéder séquentiellement, on peut mettre en présence le support, le composé inhibiteur, l'enzyme et le carbodiimide.

Le produit obtenu par le procédé selon l'invention a une activité enzymatique qui se rapproche de celle de l'enzyme libre correspondant, propriété qui est rarement conservée dans les procédés de fixation connus. De plus, ledit produit est stable pendant plusieurs mois et a une température d'inactivation plus élevée que l'enzyme libre correspondant.

Comme mentionné plus haut, ce produit peut être employé dans les réactions enzymatiques à la place de l'enzyme libre correspondant en lit fixe (colonne) ou, de préférence, en lit fluidisé. Son emploi dans l'industrie alimentaire ne doit soulever aucune difficulté, le chitosane étant une substance organique naturelle.

Les exemples suivants illustrent la mise en œuvre du procédé selon l'invention. Dans ceux-ci, les quantités et pourcentages sont pondéraux sauf indication contraire.

### Exemple 1

#### Chargement du support avec du chitosane

On dissout 8 g de chitosane dans 300 ml d'acide acétique concentré et 400 ml d'eau. On agite le mélange à 40° C pendant une heure, et on le filtre sur un tissu 106 µm pour éliminer les résidus non-dissous. On place la solution de chitosane dans un dessicateur et on ajoute 120 g d'un gel de silice (diamètre des particules $d_p$ 100−125 µm, volume des pores 1,2 ml/g, surface spécifique 320 m$^2$/g) en agitant légèrement. On laisse cette suspension sous vide jusqu'à disparition de la mousse. Après avoir laissé le mélange reposer pendant la nuit, on décante le liquide et on récupère le gel de silice enrobé de chitosane par filtration. Après un léger lavage du gel de silice avec de l'eau, on sèche le support dans une étuve sous vide à 70° C, pendant une nuit.

#### Préparation d'un support d'immobilisation stabilisé

On met dans un dessiccateur 120 g du support ci-dessus (gel de silice chargé de chitosane), avec 1600 ml d'une solution aqueuse contenant 200 ml d'une solution de glutaraldéhyde à 25%. On agite légèrement la suspension qu'on met ensuite sous vide. Après dégazage, on laisse la suspension à la pression atmosphérique pendant 2 heures, en l'agitant de temps en temps légèrement. Le support est ainsi stabilisé ce qui est visible par un changement de sa couleur de blanc au jaune-orange.

On récupère le support par filtration et on le lave soigneusement à l'eau.

#### Immobilisation d'invertase sur le support stabilisé

On prépare une solution de 15 mg d'invertase (Maxinvert 200 000 de Gist − Brocades nv) dans 100 ml d'eau et on ajuste le pH à 4 avec une solution de HCl 0,1 M. On mélange 30 ml de cette solution à 3 g de support activé humide, préparé comme ci-dessus. Après avoir agité légèrement la suspension pendant 2 heures, on récupère le catalyseur par filtration. On détermine la quantitié d'enzyme active en mesurant l'activité de l'hydrolyse du sucre de canne. Les 30 ml de solution initiale contiennent 4,5 mg d'invertase, le filtrat après fixation 0,48 mg, et le catalyseur 4,02 mg. Le rendement d'immobilisation est de 89%, une perte de l'activité pendant ce procédé n'est pas détectable.

### Exemple 2

#### Retraitement de l'invertase

On traite pendant 2 heures 15 g du catalyseur humide préparé selon l'exemple 1, et contenant 16 mg d'invertase active, avec une solution de glutaraldéhyde à 1%, en agitant faiblement le mélange et on récupère le catalyseur par filtration. Après lavage, l'activité du catalyseur mesurée comme dans l'exemple 1 correspond à 14 mg d'invertase active, soit un rendement de 87%.

### Exemple 3

#### Immobilisation d'une lactase fongique sur support stabilisé

On met dans une colonne de verre de 2,5 cm de diamètre et d'une hauteur de 20 cm 28 g de support humide, activé selon l'exemple 1. La colonne fait partie d'un système de recyclage équipé d'une pompe péristaltique réglable et d'un réservoir dont le contenu est agité par un agitateur magnétique. L'entrée de la pompe est connectée au réservoir, sa sortie est placée a la base de la colonne. La sortie à la tête de la colonne ramène le milieu au réservoir. Ce système est chargé de 120 ml d'une solution aqueuse à un pH de 4. La pompe est réglée jusqu'à ce que le support soit fluidisé à une hauteur maximale de 15 cm dans la colonne. On dissout 0,6 g de lactase d'Aspergillus niger (Rapidase, France, 200 000 unités/g) dans le réservoir, pendant que le système de recirculation fonctionne, et on laisse le support à l'état fluidisé pendant 2 heures. L'analyse de l'activité établie comme dans l'exemple 1 montre que 72% de l'activité se trouve sur le support, tandis que 20% reste dans le filtrat après filtration et lavage du catalyseur.

## Exemple 4

### Retraitement de la lactase fongique immobilisée

On mélange 10 g de la lactase humide préparée selon l'exemple 3, avec 50 ml d'une solution aqueuse contenant 5% de glutaraldéhyde, ajustée à un pH de 4. On agite ce mélange de temps en temps et on récupère le catalyseur par filtration après 2 heures. L'analyse de l'activité du catalyseur (par hydrolyse du lactose) avant le retraitement indique une activité correspondant à 21 mg de préparation de lactase (Rapidase, France, 200 000 unités par gramme) par gramme de catalyseur humide, tandis qu'après le retraitement, on mesure une activité correspondant à 19 mg par gramme de catalyseur humide. Cela correspond à une préservation de l'activité de 90%.

## Exemple 5

### Immobilisation de trypsine sur support stabilisé

On suspend 1 g de gel de silice chargé de chitosane (selon l'exemple 1) dans 20 ml de tampon acétate de sodium (0,5 M; pH 6,0). On ajoute 5 ml de glutaraldéhyde (solution aqueuse à 25%) préalablement dilué 1 : 1 par le tampon précédent. Ce traitement activateur est poursuivi pendant 30 min à température ambiante (25°C) sous légère agitation. Le matériau ainsi activé est lavé plusieurs fois à l'eau distillée pour éliminer le glutaraldéhyde résiduel. 50 mg de trypsine bovine cristallisée (Merck) sont solubilisés dans 20 ml de tampon borate de sodium (0,1 M; pH 8,0) contenant du chlorure de calcium (20 mM). On ajoute alors la solution de trypsine au gel de silice chargé de chitosane préparé auparavant. On laisse en contact une nuit à 4°C sous agitation rotative modérée. On sépare ensuite le support chargé d'enzyme de la solution par centrifugation ou filtration, puis on le lave plusieurs fois successivement par: a) du tampon borate de sodium (0,1 M; pH 8,0) contenant du chlorure de sodium (0,15 M), b) du tampon borate de sodium (0,1 M; pH 8,0) et c) de l'eau distillée jusqu'à ce que l'adsorption à 280 nm (spectromètre UV) des eaux de lavage soit nulle. L'activité enzymatique de la trypsine soluble est déterminée en utilisant comme substrat de chlorhydrate de Nα-benzoyl-L-arginine-p-nitroanilide (L-BANA) à la concentration de 1 mM, dans du tampon Tris(ou tris-hydroxyméthylamino méthane)-HCl (50 mM; pH 8,0) et à 25°C. La p-nitroaniline libérée par l'hydrolyse enzymatique est mesurée par spectrophotométrie UV à 405 nm. Une unité d'activité trypsine (1 IU) correspond à la quantité d'enzyme qui libère 1 micromole de p-nitroaniline par min dans les conditions données. L'activité de la trypsine immobilisée est déterminée selon la même méthode mais en lit agité, en utilisant un dispositif à mesure intermittente analogue à celui décrit dans »Methods in Enzymology, 44, 344 — 345 (1976)«. La quantité d'enzyme fixé sur le support est obtenue indirectement en calculant la différence entre la quantité d'enzyme ajouté initialement et la quantité d'enzyme non fixé, sur la base des concentrations en protéine déterminées par la méthode de Folin-Lowry (J. Biol. Chem. 193, 265, 1951). La préparation obtenue contient 35,2 mg de trypsine par gramme de support et a une activité enzymatique de 72 unités internationales (UI) par gramme de support. Le rendement de fixation est de 63,7%. L'activité spécifique de la trypsine immobilisée est de 2,04 UI/mg d'enzyme immobilisé alors que celle de l'enzyme soluble est de 2,26 UI/mg.

## Exemple 6

### Stabilisation du support pour l'immobilisation d'enzymes sensibles au glutaraldéhyde

40 g de support enrobé de chitosane selon l'exemple 1 sont dispersés dans 150 ml de tampon phosphate de potassium (50 mM; pH 6,5). On ajoute 1,5 ml de glutaraldéhyde (solution aqueuse à 25%, Fluka AG, pract.). On laisse réagir 30 min sous agitation rotative lente, à température ambiante. On sépare ensuite le support de la solution par filtration (tamis métallique, maille de 50 µm) ou centrifugation, puis on le lave plusieurs fois avec de l'eau distillée. On resuspend le support dans 150 ml du tampon précédent. On ajoute 500 mg de borohydrure de sodium solide, par portion. On laisse réagir 30 min dans les mêmes conditions. On récupère le support par filtration, on le lave avec de l'eau distillée, on l'essore puis l'utilise pour l'immobilisation d'enzymes comme indiqué dans les exemples 7 et 8 ci-après.

## Exemple 7

### Immobilisation d'une lactase neutre de levure par adsorption

40 g de support stabilisé selon l'exemple 6 sont dispersés dans 150 ml de tampon phosphate de potassium (50 mM; pH 6,5) contenant 0,1 mM de chlorure de manganèse et 0,02% d'azidure de sodium. On ajoute du D-galactono-γ-lactone (Koch-Light Lab.) à la concentration finale de 0,1 M. On ajuste le pH du milieu à 6,5 si nécessaire. On ajoute 6 ml d'une solution de lactase de levure (Maxilact LX 5000 de Gist-Brocades na, Delft Hollande). On agite le milieu contenant l'enzyme et le support pendant 16 h. On sépare le support du milieu par filtration et on le lave successivement par: 500 ml de tampon phosphate de potassium (50 mM; pH 6,5) et 0,1 mM de chlorure de manganèse; 500 ml de tampon précédent contenant en plus 50 mM de chlorure de potassium; de l'eau distillée (3 × 500 ml). Le support doué d'activité lactasique est conservé dans le premier tampon mentionné (phosphate-manganèse-azidure).

L'activité enzymatique soluble ou immobilisée est déterminée par spectrophotométrie UV en utilisant comme substrat une solution 5 mM d'o-nitrophényl-$\beta$-galactopyranoside (ONPG) à 30°C. L'o-nitrophénol libéré par l'enzyme est déterminé à 405 nm ($\varepsilon_M$ ONP: 1097 M$^{-1}$cm$^{-1}$ à pH 6,5). L'activité de l'enzyme immobilisé est déterminée comme indiqué dans l'exemple 5, en utilisant le substrat indiqué ci-dessus. Une unité internatione (UI) d'activité lactasique correspond à la libération de 1 micromole d'o-nitrophénol par min dans les conditions indiquées. La lactase immobilisée possède une activité de 423 UI/g de support sec.

Exemple 8

Immobilisation d'une lactase neutre
de levure par covalence

L'enzyme adsorbé selon l'exemple 7 peut être plus fortement lié à son support par un traitement additionnel au carbodiimide. Après 30 min de contact entre la lactase et son support, dans les conditions décrites dans l'exemple 7, on ajoute par petites portions 2,5 g de carbodiimide 1-cyclohexyl-3(2-morpholinoethyl) carbodiimide de métho-p-toluidine sulfonate (Sigma Chem. Co.) en maintenant le pH à 6,5 pendant 2 h à température ambiante, puis 16 h à 4°C sous agitation rotative. On procède par la suite aux mêmes lavages que ceux indiqués dans l'exemple 7. L'activité lactasique du support déterminée comme indiqué dans l'exemple 5 est de 285 UI/g de support sec.

Exemple 9

Hydrolyse de lactosérum

On pompe du lactosérum déprotéiné de bas en haut au travers de la colonne selon l'exemple 3, remplie du catalyseur retraité selon l'exemple 4, à la température de 50°C. On constate une baisse de l'activité de catalyseur de 18% pendant les premières 12 heures. Après cette baisse initiale, on constate, en effectuant une détermination de l'activité du catalyseur après une semaine, que celle-ci est stable dans les conditions du traitement indiquées ci-dessus.

Exemples 10

Hydrolyse en continu de lait écrémé

On dépose 90 ml d'une suspension de biocatalyseur selon l'exemple 8 dans une colonne de 2,5 cm de diamètre. La hauteur du lit d'enzyme est de 18,3 cm. On pompe du lait écrémé stérilisé (UHT) des bas en haut au travers de la colonne avec un débit de 180 ml/heure. Dans ces conditions, le lit d'enzyme se fluidise et atteint une hauteur de 25,5 cm. On maintient la température du système à 6,2°C et le taux d'hydrolyse du lactose est de 92%. Après 275 h d'opération continue (excepté quelques heures de lavage par un détergent approprié tous les 2–3 jours), l'activité restante du catalyseur représente les 85% de l'activité initiale.

**Revendications**

1. Procédé de préparation de biocatalyseurs doués d'activité enzymatique insolubles en milieu aqueux et convenant particulièrement à l'utilisation en lit fluidisé ou en lit fixe, caractérisé par le fait qu'on enrobe des particules d'une matière minérale rigide et dense avec une couche de chitosane, qu'on stabilise les particules enrobées par un réactif bi-fonctionnel et qu'on fixe un enzyme sur le support ainsi traité.

2. Procédé selon la revendication 1, caractérisé par le fait que le réactif bi-fonctionnel est le glutaraldéhyde.

3. Procédé selon la revendication 1, caractérisé par le fait que la matière minérale est choisie parmi les particules poreuses de gel de silice, d'oxyde métallique ou de céramique.

4. Procédé selon l'une des revendication 1 à 3, caractérisé par le fait que, l'enzyme n'étant pas sensible au glutaraldéhyde, on active le support enrobé de chitosane par traitement avec du glutaraldéhyde et on fixe l'enzyme au support par liaison chimique entre les groupes aldéhydes libres et les groupes amino libres portés par l'enzyme.

5. Procédé selon la revendication 4, caractérisé par le fait que après fixation de l'enzyme, le support est retraité par le glutaraldéhyde de manière à augmenter sa stabilité.

6. Procédé selon l'une des revendications 1 à 3, caractérisé par le fait que, l'enzyme étant sensible au glutaraldéhyde, on traite le catalyseur stabilisé par le glutaraldéhyde à l'aide d'un réactif réducteur et on adsorbe l'enzyme sur le support.

7. Procédé selon la revendication 6, caractérisé par le fait que le réactif réducteur est le borohydrure de sodium.

8. Procédé selon la revendication 6, caractérisé par le fait que l'enzyme adsorbé est lié chimiquement au support par un agent de couplage, de préférence un carbodiimide ou le réactif de Woodward.

9. Procédé selon la revendication 8, caractérisé par le fait que les sites actifs de l'enzyme sont protégés par adjonction d'un inhibiteur compétitif.

10. Procédé selon la revendication 9, caractérisé par le fait que l'inhibiteur compétitif est la glucono-$\gamma$-lactone ou la galactono-$\gamma$-lactone.

11. Biocatalyseur préparé par le procédé selon l'une des revendications 1 à 10.

## Patentansprüche

1. Verfahren zur Herstellung von eine enzymatische Aktivität aufweisenden Biokatalysatoren, die in einem wäßrigen Milieu unlöslich sind und insbesondere für eine Verwendung in einem Fließbett oder in einem Festbett geeignet sind, dadurch gekennzeichnet, daß man Teilchen aus einem starren und dichten mineralischen Material mit einer Chitosan-Schicht ummantelt, daß man die ummantelten Teilchen durch ein bifunktionelles Reagens stabilisiert und daß man auf dem so behandelten Träger ein Enzym fixiert.

2. Verfahren nach Anspruch 1, dadurch gekennzeichnet, daß das bifunktionelle Reagens Glutaraldehyd ist.

3. Verfahren nach Anspruch 1, dadurch gekennzeichnet, daß das mineralische Material ausgewählt ist aus porösen Teilchen aus Kieselgel, aus einem Metalloxid und aus einer Keramik.

4. Verfahren nach einem der Ansprüche 1 bis 3, dadurch gekennzeichnet, daß man dann, wenn das Enzym gegen Glutaraldehyd nicht empfindlich ist, den mit Chitosan ummantelten Träger durch eine Behandlung mit Glutaraldehyd aktiviert und daß man das Enzym durch eine chemische Bindung zwischen den freien Aldehydgruppen und den freien Aminogruppen am Enzym am Träger fixiert.

5. Verfahren nach Anspruch 4, dadurch gekennzeichnet, daß man den Träger nach der Fixierung des Enzyms mit Glutaraldehyd nachbehandelt, so daß seine Stabilität erhöht wird.

6. Verfahren nach einem der Ansprüche 1 bis 3, dadurch gekennzeichnet, daß man dann, wenn das Enzym gegen Glutaraldehyd empfindlich ist, den mit Glutaraldehyd stabilisierten Katalysator mit einem Reduktionsmittel behandelt und man dann das Enzym an dem Träger adsorbiert.

7. Verfahren nach Anspruch 6, dadurch gekennzeichnet, daß das Reduktionsmittel Natriumborhydrid ist.

8. Verfahren nach Anspruch 6, dadurch gekennzeichnet, daß das adsorbierte Enzym chemisch durch ein Kupplungsmittel am Träger gebunden ist, vorzugsweise durch ein Carbodiimid oder das Woodward-Reagens.

9. Verfahren nach Anspruch 8, dadurch gekennzeichnet, daß die aktiven Stellen des Enzyms durch Anfügen eines konkurrierenden Inhibitors geschützt sind.

10. Verfahren nach Anspruch 9, dadurch gekennzeichnet, daß der konkurrierende Inhibitor Glucono-$\gamma$-Lacton oder Galactono-$\gamma$-Lacton ist.

11. Biokatalysator, der nach einem der Ansprüche 1 bis 10 hergestellt wurde.

## Claims

1. A process for the production of an enzymatically active biocatalyst insoluble in aqueous medium and particularly suitable for use in fluidised-bed or fixed-bed reactors, which comprises coating particles of a rigid and dense mineral material with a layer of chitosan, stabilising the coated particles with a bifunctional reagent and fixing an enzyme to the support thus treated.

2. A process as claimed in Claim 1, wherein the bifunctional reagent is glutaraldehyde.

3. A process as claimed in Claim 1 or 2, wherein the mineral material is porous particles of silica gel, metal oxides or ceramics.

4. A process as claimed in any of Claims 1 to 3, wherein the enzyme is not sensitive to glutaraldehyde and the support coated with chitosan is activated by treatment with glutaraldehyde and the enzyme is fixed to the support by chemical binding between the free aldehyde groups and the free amino groups carried by the enzyme.

5. A process as claimed in Claim 4, wherein after fixing of the enzyme, the support is retreated with glutaraldehyde to increase its stability.

6. A process as claimed in any of Claims 1 to 3, wherein the enzyme is sensitive to glutaraldehyde and the catalyst stabilised with glutaraldehyde is treated with a reducing reagent and the enzyme is adsorbed onto the support.

7. A process as claimed in Claim 6, wherein the reducing reagent is sodium borohydride.

8. A process as claimed in Claim 6, wherein the adsorbed enzyme is chemically bound to the support by a coupling agent, preferably a carbodiimide or Woodward's reagent.

9. A process as claimed in Claim 8, wherein the active sites of the enzyme are protected by the addition of a competing inhibitor.

10. A process as claimed in Claim 9, wherein the competing inhibitor is glucono-$\gamma$-lactone or galactono-$\gamma$-lactone.

11. An enzymatically active biocatalyst as produced by the process of any of Claims 1 to 10.